# EUROPEAN PATENT SPECIFICATION

(11) **EP 3 534 915 B1**
(45) Date of publication and mention of the grant of the patent: **20.12.2023**
(21) Application number: 17809399.3
(22) Date of filing: 31.10.2017
(51) Int. Cl.: A23L 33/165, A61K 9/00, A61K 9/28, A61K 9/50, A61K 31/315, A61K 47/34, A61K 47/64, A61K 47/69, A61P 1/00, A61P 1/04, A61P 1/14, A61P 3/02, A61P 15/00, A61P 15/10, A61P 17/02, A61P 25/02, A61P 25/14, A61P 37/02, A61K 33/30, A61K 9/20

(54) **GAMMA-POLYGLUTAMIC ACID AND ZINC COMPOSITIONS**
GAMMA-POLYGLUTAMINSÄURE UND ZINK PRÄPARATE
COMPOSITIONS COMPRENANTS DE L'ACIDE POLY-L-GLUTAMIQUE ET DU ZINC

(30) Priority: 01.11.2016 SG 10201609137P
(43) Date of publication of application: 11.09.2019
(73) Proprietor: Xylonix Pte. Ltd., Singapore 079903 (SG)
(72) Inventor: CHUNG, Jinhyuk Fred, Singapore 437522 (SG)
(74) Representative: Hoffmann Eitle
(86) International application number: PCT/SG2017/050544
(87) International publication number: WO 2018/084805

(56) References cited:
- WO-A1-2004/080210
- IT-A1- MI 950 515
- US-A1- 2005 100 593
- DATABASE WPI Week 200748 Thomson Scientific, London, GB; AN 2007-496073 XP002777516, -& WO 2007/043606 A1 (BIOLEADERS CORP) 19 April 2007 (2007-04-19)
- SUBARNA KARMAKER ET AL: "A Zinc(II)/Poly( [gamma] -glutamic acid) Complex as an Oral Therapeutic for the Treatment of Type-2 Diabetic KKA y Mice", MACROMOLECULAR BIOSCIENCE, vol. 9, no. 3, 10 March 2009 (2009-03-10), pages 279-286, XP055439227, DE ISSN: 1616-5187, DOI: 10.1002/mabi.200800190

## Description

### FIELD OF THE INVENTION

The invention relates to the field of nutritional supplements, and more particularly relates to a composition comprising gamma-polyglutamic acid and a zinc salt in a solid or liquid oral dosage form supplement to provide zinc to a human being desiring or in need thereof, including human beings with a zinc deficiency or zinc inadequacy.

### BACKGROUND OF THE INVENTION

Zinc is an essential mineral for microorganisms, plants, and animals, including human beings. Zinc is necessary for numerous catalytic, structural, and regulatory functions in the body. Nearly 100 metalloenzymes require zinc for catalytic activity, and these metalloenzymes are distributed amongst all six enzyme classes. Zinc also binds to proteins to influence the protein structure. In some cases proteins fold into a particular structure as a result of zinc coordinating to certain amino acids, and the folded protein is biologically active. In other cases, zinc coordination influences the catalytic activity of another metal in a metalloenzyme by altering the structure of the enzyme. Zinc has also been identified as a regulator of gene expression, and acts by binding to, e.g., metal response element transcription factor.

Because there is no mechanism for storing zinc, a regular intake of zinc is needed to maintain adequate levels of the mineral in the body. Zinc is generally obtained through the diet. Food sources for zinc include red meat, poultry, seafood such as crab and lobster, beans, nuts, and dairy products. However, phytates (e.g., myo-inositol hexaphosphate), present in foods such as whole-grain breads, cereals, and legumes, bind zinc and thus reduce the absorption of zinc in the gastrointestinal tract. Dietary imbalances can lead to inadequate zinc levels, and in particular, diets rich in phytates have been linked to zinc deficiency in some populations. Deficiencies may also occur in individuals with impaired zinc absorption due to, for example, sprue, Crohn's disease, short bowel syndrome, or an inherited defect in a zinc carrier protein.

It is estimated that 25% of the world population is at risk for zinc deficiency. Maret W, Sandstead HH, "Zinc requirements and the risks and benefits of zinc supplementation," J. Trace Elem. Med. Biol. 20 (1): 3-18 (2006). Zinc inadequacy or deficiency is linked to impaired immune function, loss of appetite, delayed wound repair, stunted growth, impotence, delayed puberty, taste abnormalities, and behavioral abnormalities, and other conditions.

To minimize the chance of inadequate or deficient zinc levels, some foods are fortified with zinc, such as cereals, and zinc may be added to soil to produce crops with increased zinc content. Nutritional (dietary) supplements are also available. Such supplements provide zinc in the form of, for example, zinc sulfate, zinc acetate, or chelated zinc, and there are topical agents containing zinc oxide. However, there are drawbacks to the zinc supplements currently on the market because they directly introduce the zinc salt to the stomach. Zinc salts introduced into the stomach are known to cause stomach irritation or even gastric bleeding. Zn²⁺ ions compete with other divalent metal ions, such as calcium, magnesium, copper, and iron, for absorption in the gastrointestinal tract. It is reported that high zinc intake, such as might occur with a supplement, can inhibit copper absorption and lead to copper deficiency or anemia. Willis MS, Monaghan SA, Miller ML, McKenna RW, Perkins WD, Levinson BS, et al. "Zinc-induced copper deficiency: a report of three cases initially recognized on bone marrow examination," Am. J. Clin. Pathol. 123:125-31 (2005). To properly intake zinc from a supplement it needs to be taken more than two hours apart from meals with foods that bind zinc, or from supplements for other nutrients, such as copper, iron, or phosphorus, that compete in the natural absorption mechanism or bind zinc.

Accordingly, there remains a need for zinc supplement compositions that avoid the problems associated with currently available compositions, in particular compositions that avoid stomach upset and gastric irritation, and that also avoid competition among divalent metal ions for bioabsorption in the gastrointestinal tract.

US20050100593 discloses a capsule film composition comprising gelatin and poly glutamic acid. The capsule content ingredient may be a mineral, such as calcium, iron, zinc, copper or magnesium. The capsule preparations accelerate absorption of minerals such as calcium and can easily be administered orally.

WO2007043606 discloses an agent for treating diabetes capable of being administered orally, comprising poly-gamma-glutamic acid and a metal compound.

SUBARNA KARMAKER ET AL: "A Zinc(II)/Poly-gamma-glutamic acid Complex as an Oral Therapeutic for the Treatment of Type-2 Diabetic KKA y Mice", MACROMOLECULAR BIOSCIENCE, vol. 9, no. 3, 10 March 2009, pages 279-286, discloses a new insulin mimetic zinc(II)/poly-gamma -glutamic acid complex.

### SUMMARY OF THE INVENTION

Nutritional supplement compositions for providing zinc to a person in need thereof that overcomes the problems and side effects recognized in the art are provided. The invention is directed to compositions for administering zinc comprising gamma-polyglutamic acid (y-PGA), a zinc salt, and a gastro-resistant material, formulated as a solid or liquid dosage form.

Without being limited by theory, the invention contemplates providing a solid or liquid dosage form having a gastro-resistant material, such that the contents of the dosage form are not released until the dosage form passes through the stomach and enters the intestine. In the intestine, the invention contemplates presenting a zinc salt in the presence of γ-PGA such that zinc may be absorbed by being associated and transported with γ-PGA in the large intestine and not necessarily via the transport mechanism for divalent metal ions operative in the small intestine.

In an embodiment, the composition comprises a formulation comprising γ-PGA, having an average molecular weight in the range of about 5 kDa to about 300 kDa, a zinc salt, and a gastro-resistant material.

In an embodiment, the composition comprises γ-PGA and a zinc salt contained in a solid or liquid dosage form, and means for delivering said γ-PGA and said zinc salt to the intestine. The corresponding structure or material for performing the function of delivering said components to the intestine is provided by a gastro-resistant material as part of the solid dosage form or as part of suspended solids, e.g., granules, particles, and the like, in the liquid dosage form, respectively.

In combination with any of the above embodiments for solid dosage forms, the compositions are prepared as a solid dosage form, such as a capsule or a tablet, and may also comprise one or more excipients selected from fillers and/or binders and/or granulating agents and/or compression aids and/or disintegrants, and may also further comprise lubricants and/or glidants.

The invention is also directed to methods for preparing a solid dosage form of the nutritional supplement compositions disclosed herein. In an embodiment, the solid dosage form is a tablet. In another embodiment, the solid dosage form is a capsule.

In one embodiment the method comprises mixing γ-PGA and a zinc salt, and one or more excipients, granulating the mixture, and mixing a lubricating agent and optionally, additional excipient(s) with the granulated mixture, tableting the obtained composition into tablets, and coating the tablets with a gastro-resistant substance. In one embodiment the method comprises mixing γ-PGA and a zinc salt, and one or more excipients, granulating the mixture, and optionally, mixing additional excipient(s) with the granulated mixture, and then enclosing the obtained composition in a capsule, and the capsule comprises or is provided with a gastro-resistant coating.

In one embodiment, the method comprises mixing γ-PGA and a zinc salt, a gastro-resistant binder and one or more excipients, granulating the mixture, and mixing a lubricating agent and optionally, additional excipient(s) with the granulated mixture, tableting the obtained composition into tablets, and optionally coating the tablets for reasons of appearance, mechanical stability, and the like. In one embodiment the method comprises mixing γ-PGA and a zinc salt, a gastro-resistant binder, and one or more excipients, granulating the mixture, and optionally, mixing additional excipient(s) with the granulated mixture, and then enclosing the obtained composition in a capsule.

In one embodiment the method comprises mixing γ-PGA and a zinc salt, and one or more excipients, to form a solid mixture, such as a powder. In some embodiments, the solid mixture composition is formulated as a suitable dosage form, such as a tablet or a capsule, and the like.

The invention is also directed to methods for preparing a liquid dosage form of the nutritional supplement compositions disclosed herein, as a liquid suspension.

In one embodiment, the method comprises mixing γ-PGA and a zinc salt, a gastro-resistant binder and one or more excipients, granulating the mixture, and then suspending the granulated solid in a suitable liquid. Methods for providing zinc to a person desiring or in need thereof are also provided. In one embodiment, the method comprises administering to a human being desiring or in need of nutritional supplementation of zinc the solid or liquid dosage form of the nutritional supplement compositions disclosed herein. In some embodiments, the method provides up to 100 mg, up to 75 mg, up to 50 mg, up to 25 mg, or up to 10 mg of zinc in the solid dosage form(s) administered.

These and other objects and features of the invention will become apparent to one of ordinary skill in the art from the following detailed description of the invention and are limited by the subject-matter of the appended claims.

### DETAILED DESCRIPTION OF THE INVENTION

The components used in the compositions and formulations described herein are of a grade accepted by regulatory authorities for use in nutritional supplement product, and in some instances means pharmaceutical grade or medical grade compounds or substances.

The meaning of abbreviations used herein is as follows: "kDa" means kiloDalton; "wt%" means percent by weight.

### Active Ingredients

Zinc is provided as a zinc(II) salt (equivalently, a Zn²⁺ salt), wherein the counterion (anion) may be any suitable inorganic or organic anion. Suitable anions are those that are tolerated by the human body, including those that are not toxic. Generally, the zinc salt can be represented by the formulas Zn²⁺X²- or Zn²⁺(X⁻)₂ or even Zn²⁺(X⁻)(Y⁻), where X and Y are suitable anions. The anion may be selected from the group of anions that are a component of an approved food additive or nutritional supplement. In some embodiments, the zinc(II) salt is a nutritionally acceptable zinc salt, wherein said zinc(II) salt is selected from the group of zinc(II) salts that have been approved for use in foods or nutritional supplements. The anion may be selected from the group of anions that are a component of an FDA-approved pharmaceutical product. In some embodiments, the zinc(II) salt is a pharmaceutically acceptable zinc salt. Examples of zinc salts include zinc chloride, zinc sulfate, zinc citrate, zinc acetate, zinc picolinate, zinc gluconate, amino acid-zinc chelates, such as zinc glycinate, or other amino acids known and used in the art. Two or more different zinc salts may be used together in any proportion for providing Zn(II) in any of the compositions or formulations.

The amount of zinc included in a single dosage form is generally in the range of about 1 to about 100 mg of zinc (zinc(II) ion). Thus, the particular amount of zinc salt(s) used in a formulated composition will be higher because amount of the salt must account for the weight of the counterion. Considering only zinc(II), the amount provided in a dosage form may be up to about 100 mg, up to about 75 mg, up to about 50 mg, up to about 25 mg, up to about 10 mg of zinc, or up to about 5 mg. The amount of zinc(II) provided is generally at least about 1 mg. Commonly available supplements provide, for example, 20, 25, 30, 50, 75, and even 100 mg of zinc. Any amount of zinc in this range, or even higher, is acceptable so long as the amount provided does not cause excessive levels of zinc to be absorbed. Although a tolerable upper intake level of zinc in most adults is about 40 mg/day (and for children it is lower), it should be recognized that all of the zinc in the dosage form is unlikely to be absorbed; some of it will pass through the body without being adsorbed. Because the amount of zinc absorbed will also vary with the formulation, the upper limit for zinc content in a particular formulation can be tested by methods known in the art to ascertain the level of uptake provided by the formulation and adjust the upper limit for dosage form accordingly.

Gamma-polyglutamic acid (alternatively γ-polyglutamic acid or γ-PGA) is a polymer of glutamic acid, an amino acid, where the polymer backbone is formed by a peptide bond joining the amino group and carboxyl group in the amino acid side chain (at the γ-carbon). γ-PGA can be formed from the L isomer, the D isomer, or the DL racemate of glutamic acid. Any of these forms may be used, and two or more different forms may be used together in any proportion. The various isomeric forms of γ-PGA may be synthetic or derived from natural sources. γ-PGA is found, for example, in Japanese natto and in sea kelp. Whereas organisms usually only produce poly(amino acids) from the L isomer, certain bacterial enzymes that produce γ-PGA can produce polymers from either isomer or both isomers.

γ-PGA of various sizes and various polymer dispersities may be used. The polymer molecular weight of γ-PGA is generally at least about 5 kDa and at most about 300 kDa. In some embodiments, the polymer molecular weight of γ-PGA is at least about 5 kDa, or least about 10 kDa, or at least about 20 kDa, or least about 30 kDa, or at least about 35 kDa, or at least about 40 kDa, or at least about 50 kDa. In some embodiments, the polymer molecular weight of γ-PGA is at most about 300 kDa, or at most about 200 kDa, or at most about 100 kDa. An acceptable polymer molecular weight range may be selected from any of the above indicated polymer molecular weight values. In an embodiment, the polymer molecular weight is in the range of about 5 kDa to about 300 kDa. In an embodiment, the polymer molecular weight is in the range of about 50 kDa to about 100 kDa. In one embodiment, the polymer molecular weight is about 100 kDA. In one embodiment, the polymer molecular weight is about 50 kDA.

Polymer molecular weights are typically given as a number average molecular weight (Mₙ) based, for example, on a measurement by gel permeation chromatography (GPC). The above polymer masses are cited as Mₙ; other measurement techniques can be used to determine, e.g., a mass (weight) average molecular weight (M_{w}), and the specification for any given polymer can be converted among the various polymer mass representations.

The amount of γ-PGA included in a solid dosage form is generally in the range of about 10 wt% to about 40 wt%. In some embodiments the amount is about 20 wt% or about 30 wt%. The amount used is generally based upon the desired molar ratio between zinc and polyglutamic acid monomer units, the mass of the zinc salt (accounting for the weight of the counterion), and the amount of excipients needed to provide an acceptable formulated dosage form. For example, the greater the amount of γ-PGA and zinc salt used, the lesser the amount of excipients that can be added for a given overall dosage form size. Those of skill in the art can readily balance the amount of active ingredients versus the amount and type of excipients needed to obtain stable dosage forms. The desired ratio between zinc and γ-PGA can also be expressed as a ratio of milligrams of zinc to wt% of γ-PGA per dosage form. Exemplary ratios include 5 mg:10 wt%; 5 mg: 20 wt%; 5 mg: 40 wt%; 30 mg:10 wt%; 30 mg: 20 wt%; 30 mg: 40 wt%; or even 100 mg:10 wt%; 100 mg: 20 wt%; 100 mg: 40 wt%; or any other sets of values within the ranges set forth by these exemplary ratios or that are apparent from the values cited for each ingredient in this specification.

The amount of γ-PGA included in a liquid dosage form is generally in the range of about 0.01 wt% to about 10 wt%. In some embodiments the amount is about 0.1 wt% or about 1 wt%. The amount used is generally based upon the desired molar ratio between zinc and polyglutamic acid monomer units.

A gastro-resistant material is included as a gastro-resistant outer coating or alternatively as a gastro-resistant binder, and is considered an active ingredient in the compositions and formulated dosage forms for the purposes of this disclosure. The material that makes up the gastro-resistant outer coating or binder serves the function of delaying the release of zinc salt and γ-PGA from the dosage form until it passes through the stomach and enters the intestine. Generally, a gastro-resistant material is a matrix or polymer or other barrier that does not appreciably dissolve or swell in the acidic environment (pH ~3) of the stomach, but will dissolve or swell enough that the contents are released in the neutral to slightly alkaline environment (pH 7-9) of the intestine. Enteric coatings and enteric binders are examples of a gastro-resistant material.

Examples of gastro-resistant materials include cellulose acetate phthalate, cellulose acetate succinate, cellulose acetate trimellitate, hydroxypropylmethylcellulose-phthalate, a copolymer of two or more monomers selected from (i) an acrylate ester, (ii) a methylacrylate ester, and (iii) methacrylic acid, polyvinyl acetate phthalate, hypromellose acetate succinate, hypromellose phthalate, sodium alginate, shellac, and zein.

Numerous grades and pharmacopeial standards exist for gastro-resistant materials, and they provide a useful guide to selecting a suitable material for providing the function of delivering zinc and γ-PGA to the intestine. By controlling the coating thickness and polymer composition in an outer coating, or the amount of binder and the polymer composition, the release point can be adjusted to occur earlier or later, or within certain approximate regions of the intestine. Examples of the degree of control that can be achieved can be found in the line of methacrylic acid co-polymers available from Corel Pharma Chem (India) under the trade name Acrycoat^{®} that meet various pharmacopeial standards, such as: USP/NF methacrylic acid copolymer, type A-NF, used at 4-5% and typically delivers the dosage form contents to the jejunum; USP/NF methacrylic acid copolymer, type C-NF, used at 4-5% and typically delivers the dosage form contents to the duodenum; and USP/NF methacrylic acid copolymer, type B-NF, used at 10-20% and typically delivers the dosage form contents to the colon. The latter (type B-NF) achieves the delivery with a pH-dependent polymer, though pH-independent polymers also can be used for delivery to the colon or the intestine as well.

### Formulation

The zinc salt and γ-PGA active ingredients can be formulated into oral solid dosage forms such as a tablet, a capsule, or related forms for oral administration such as a minitablet, a caplet, and the like. The dosage form may include a gastro-resistant binder, or may be further formulated to have a gastro-resistant outer coating.

The zinc salt and γ-PGA active ingredients can be formulated into oral liquid dosage forms such as a liquid suspension. The formulation components and method of preparing embodiments of a liquid dosage form are described further below.

The zinc salt and γ-PGA active ingredients are combined with excipients suitable for use in a supplement product and suitable for making a particular solid dosage form, such as a tablet or a capsule, and the like. Typical excipients include fillers, binders, disintegrants, glidants, lubricants, as well as buffers, preservatives, anti-oxidants, flavoring agents, sweeteners, coloring agents, and the like. The amount and type of excipient to be added can be selected for various purposes, such as improved integrity of the dosage form, improved bioavailability, stability, manufacturing, coating, appearance, and/or compliance. Some excipients may serve more than one purpose and/or provide more than one improved characteristic.

Fillers may be water soluble or water insoluble, and one or more of each type may be combined. Examples of water soluble fillers include, without limitation, sugars such as glucose, fructose, sucrose, mannose, dextrose, galactose, and the like, and sugar alcohols, such as mannitol, sorbitol, xylitol, and the like, as known in the art. Examples of water insoluble fillers include, without limitation, waxes, long-chain fatty acids, talc, kaolin, silicon dioxide, titanium dioxide, alumina, starch, powdered cellulose, microcrystalline cellulose, and the like, as known in the art.

Binders include, without limitation, cellulose derivatives such as carboxymethylcellulose calcium, carboxymethylcellulose sodium, cellulose acetate phthalate, ethyl cellulose, hydroxyethyl cellulose, hydroxyethylmethyl cellulose, hydroxypropyl cellulose, hydroxypropyl methyl cellulose, methyl cellulose, microcrystalline cellulose, polyvinyl pyrrolidone, as well as starches, modified starches, such as partially hydrolyzed starch, e.g., maltodextrin, saccharides, gelatin, natural or synthetic gums, and the like, as known in the art.

As described above, in some embodiments, gastro-resistant binders may be included in the formulation as a binder that functions to delay the release of the zinc(II) salt and γ-PGA until the intestine. When a gastro-resistant binder is used, it may be used in combination with other (non-gastro-resistant) binders.

Disintegrants include, without limitation, carmellose, carmellose sodium, croscarmellose sodium, crospovidone, alginates, low substituted hydroxypropyl cellulose, hydroxypropyl starch, partially pregelatinized starch, and the like, as known in the art.

Glidants include, without limitation, silicas, silicates, talc, calcium phosphate, and the like, as known in the art.

Lubricants include, without limitation, alkali metal or alkaline earth metal stearates, oleates, benzoates, acetates, chlorides, and the like, as known in the art.

Other types of excipients, such as buffers, preservatives, anti-oxidants, flavoring agents, sweeteners, coloring agents, are well-known and persons of ordinary skill in the art can readily select and apply such components to the formulations.

Other types of active ingredients, such as vitamins, minerals, nutrients, and other nutritional or dietary supplements that are amenable to absorption in the intestine may also be added to the solid or liquid compositions and formulations described herein without departing from the scope of the invention, unless stated otherwise.

The solid or liquid compositions and formulations described herein may alternatively comprise, consist of, or consist essentially of zinc salt(s) and γ-PGA and a gastro-resistant outer coating or a gastro-resistant binder, so long as it is consistent with the specification. The solid or liquid compositions and formulations may also lack or be substantially free of any component(s), e.g. active ingredient and/or excipient found in a prior art composition or that are otherwise not necessary to the disclosed invention.

### Methods of Preparing Solid Dosage Forms

The zinc salts and γ-PGA, and the selected excipients may be sized, declumped, or powderized individually or in combination. The various components may be combined by dry mixing, or granulated by wet or dry granulation, spray, extrusion, rolling, or fluidized bed granulation, and thereafter may optionally be milled, or other such techniques as known in the art.

The method for preparing solid dosage forms involves mixing together the desired amounts of zinc salt(s) and γ-PGA, and the excipients, which comprise one or more filler and/or one or more binder and/or one or more disintegrant and/or one or more lubricating agent and/or one or more glidant. As described above, in some embodiments, said one or more binder may be a gastro-resistant binder, and it may be used in combination with other (non-gastro-resistant) binders. When a granulating step is included, then any of the excipients may be added, in whole or in part, before, during, or after the granulating step. In some embodiments some or all of a lubricating agent are mixed in after a granulating step. In some of these embodiments, a glidant is also mixed in after the granulating step.

Where the granulation step involves using a solvent, such as water, or an organic solvent, or an aqueous organic solution to wet the blend of components as they are granulated, the resulting product is usually dried to remove residual solvent. Examples of organic solvents include ethanol and isopropanol, and the like, as known in the art. Preferably, substantially all of an organic solvent is removed in a drying step. When water is part of the solvent used in a granulation step, preferably no more than 10 wt%, or no more than 5 wt%, or no more than 2 wt% of the water is left after drying and proceeding to the next step.

The mixed or granulated solids may be formed into tablets by tableting the solids using compression, compaction, or molding. Thereafter, in some embodiments, the tablets are coated with a gastro-resistant coating, as described above. Generally, the gastro-resistant substance and, optionally, other excipients (e.g., plasticizer, emulsifier) are dissolved or dispersed into an aqueous or organic solvent and then applied using any of numerous methods known in art, including spray coating, fluidized bed coating, pan coating, and the like. In some embodiments, the tablets are coated for purposes of appearance, mechanical stability, chemical stability, and the like, but without a gastro-resistant material included in the coating.

Alternatively, the mixed or granulated solids may be filled into a capsule or caplet, and enclosed inside. The term capsule includes soft capsules, hard capsules, gelcaps, vegetable capsules, and may be one-piece or two-piece capsules. Enterically-coated capsules are available (e.g., enteric capsule drug delivery technology), or the capsules may filled, enclosed, and then coated with the gastro-resistant coating by the methods mentioned above using a solution or dispersion of the substance, optionally with other excipients. In other embodiments, the mixed or granulated solids comprise a gastro-resistant binder material, and such solids can be loaded in capsules lacking an enteric coating.

The size and shape of either tablets or capsules is not particularly limited. It is expected that the desired dosage amounts of zinc salts and γ-PGA can be formulated into a tablet or capsule that is not unduly large.

Exemplary methods for preparing tablet dosage forms according to embodiments of the invention are provided below in Examples 2 and 4.

### Methods of Preparing Liquid Dosage Forms

One method for preparing liquid dosage forms comprises mixing together the desired amounts of zinc salt(s) and γ-PGA and gastro-resistant binder, along with suitable excipients to prepare a granulated solid or other solid form suitable for suspension in a solution.

Suitable excipients include fillers, binders, disintegrants, as well as buffers, preservatives, anti-oxidants, and the like (as described above with respect to solid dosage forms). The amount and type of excipient to be added can be selected for various purposes, such as improved integrity of the dosage form, improved bioavailability, stability, manufacturing, appearance, and/or compliance. Some excipients may serve more than one purpose and/or provide more than one improved characteristic.

The zinc salts and γ-PGA and gastro-resistant binder, and the selected excipients may be sized, declumped, or powderized individually or in combination. The various components may be granulated by wet or dry granulation, spray, extrusion, rolling, or fluidized bed granulation, and thereafter may optionally be milled, or other such techniques as known in the art. In particular, granulation steps may be performed as described above with regard to solid dosage forms. Thus, granulated compositions comprising mixtures of a Zn(II) salt and a γ-PGA active ingredient are prepared with a gastro-resistant binder included in the granulated solid. γ-PGA has an average molecular weight in the range of about 5 kDa to about 500 kDa. In some embodiments the average polymer molecular weight is about 5 kDa to about 100 kDa, and in other embodiments is about 1 kDa to about 100 kDa.

The granulated solid is then suspended in an acidic liquid suitable for ingestion. Such liquid may be an acidic liquid food or a liquid nutritional supplement. The pH of the solution may be less than about pH 6 so that the granulated solid remains stable as a result of the gastro-resistant binder. In one embodiment the liquid suspension formulation also contains a thickening agent or viscosity enhancer, such that the granulated solids can remain suspended sufficiently and be efficiently ingested from the container. For example, in an embodiment, after suspending the solids by shaking or stirring as necessary, the solids should have a settling time of more than about 5 seconds, or more than about 15 seconds. Other excipients may be included in the liquid suspension, such as flavoring agents, buffers, preservatives, anti-oxidants, sweeteners, and the like.

When liquid dosage forms are constituted for use, γ-PGA generally is present in a concentration of about 0.01 wt% to about 10 wt%, and the Zn(II) salt is generally present in a concentration of about 0.001 wt% to about 10 wt%.

Another method for preparing liquid dosage forms comprises forming particles, such as microspheres, microparticles, granules, or other suitable solid form of a zinc salt and γ-PGA complex, and coating the particle with a thin layer of wax. In preferred embodiments the particles further comprise a gastro-resistant binder. The coated particles are formulated as a liquid suspension formulation. The wax coating on the particles promotes physical integrity of the particle and reduces permeability, though the coating nonetheless permits delivery of the zinc and γ-PGA complex to the intestine.

Granules suitable for coating may be prepared according to any of the aforementioned methods. Microspheres or microparticles of a zinc salt, γ-PGA, and a gastro-resistant binder may be prepared by any of the numerous methods known in the art, which include the single emulsion method, double emulsion method, polymerization, interfacial polymerization, phase separation and coacervation, spray drying, spray congealing, solvent extraction, freeze drying of a dispersed phase. The dimensions of such microspheres or microparticles may range from tenths of a micron to thousands of microns. As an example, one method for preparing microspherical particles involves stirring a finely divided (e.g., powdered) solid mixture comprising a zinc salt and γ-PGA in a suspension medium such as paraffin oil, and adding a solution of a polymeric gastro-resistant binder to the stirred suspension. When the microspheres have formed a non-solvent, such as chloroform, is added to precipitate the microspheres, which are collected, dried, and subsequently coated with a wax.

Wax coatings are recognized to be biocompatible and non-immunogenic, and suitable for the entrapment and delivery of drugs to the intestinal tract. Particles (microspheres, microparticles, granules, and the like) may be coated with waxes, such as Carnauba wax, beeswax, cetostearyl alcohol, spermaceti, and other waxes, according to methods as known in the art. For example, particles may be coated with Carnauba wax by dissolving the wax in white paraffin oil, cooling the solution to less than 45°C, and then adding the particles to a mechanically-stirred wax/paraffin oil solution until the particles are coated. The stirring speed and time, and temperature of the wax solution can be adjusted to modify the thickness of the wax coating.

The wax-coated zinc salt and γ-PGA particles are formulated as a liquid suspension for administration. The coated zinc/ γ-PGA particles are present at about 5 wt% to 30 wt% in the final formulated suspension. Typically, the liquid suspension formulation comprises a suspending polymer, a viscosity agent, and a buffer. The formulation may also further comprise one or more of a sweetener, a flavoring agent, and/or a preservative.

A suspending polymer may be selected from xanthan gum, carbomer, microcrystalline cellulose, carboxymethylcellulose, and sodium carboxymethylcellulose, which may be used singly or in any combination. Other similar agents as known in the art may also be used. In total, the suspending polymer component is present at about 0.02 wt% to about 5 wt% in the final formulation.

A viscosity agent may be selected from glycerin, hydroxylpropyl cellulose, hydroxypropyl methylcellulose, povidone, guar gum, and locust bean gum, which may be used singly or in any combination. Other similar agents as known in the art may also be used. In total, the viscosity agent component is present at about 0.05 wt% to about 50 wt% in the final formulation.

A buffer may be selected from phosphate buffer, an acetate buffer, a lactate buffer, and a citrate buffer, or other nutritionally acceptable buffer that has a buffering capacity in the designated range. The buffering agents are adjusted to have pH of about 6 or lower. In some embodiments, the pH is between about 3 and about 6. In some embodiments, the pH is between 4.5 and 5, in other embodiments the pH is between 4 and 5, and in yet other embodiments the pH is between 3 and 5.

A sweetener may be selected from sucrose, invert sucrose, xylitol, sorbitol, maltitol, aspartame, saccharine, and sucralose, which may be used singly or in any combination. Other similar agents as known in the art may also be used. In total, the sweetener component may be present from about 5 wt% to 40 wt% in the final formulation.

A flavoring agent may be selected from any pharmaceutically acceptable flavoring agent, or any agent used in foods or supplements as known in the art, and may be added in amounts in the final formulation that are consistent with industry practice.

A preservative may be selected from sodium benzoate, methyl paraben, propyl paraben, benzyl alcohol, potassium sorbate, and citric acid, which may be used singly or in any combination, and may be added in amounts in the final formulation that are consistent with industry practice. Other similar agents as known in the art may also be used.

An exemplary formulation and a method for preparing a liquid dosage form according to some embodiments of the invention are provided below in Examples 5 and 6, respectively.

### Dosing and Administration

The oral dosage forms described herein may be administered to provide an effective amount of zinc to human beings who have a need or desire to supplement the zinc taken in by their diet. An effective amount means that the amount of zinc absorbed by the human being is sufficient to alleviate a zinc inadequacy or deficiency. Achieving an effective amount will depend on the formulation characteristics, and will vary by gender, age, condition, and genetic makeup of each individual. Individuals with inadequate zinc due to, for example, genetic causes or other causes of malabsorption or severe dietary restriction general are administered an effective amount by using higher dosage strength formulations or multiple dosage forms of lower strength, generally up to about 100 mg zinc per day. Individuals deficient in zinc may be administered an effective amount by using one or multiple dosage forms per day, up to, for example about 25 mg, or 50 mg, or 75 mg zinc per day. Multiple dosage forms may be taken together or separately in the day. The oral dosage forms may be administered without regard to meal time.

### EXAMPLES

### Example 1: γ-Polyglutamic acid-Zinc Composition

The composition of an exemplary embodiment of the invention is shown in Table 1. The formulation provides 25 mg of Zn (Zn²⁺ ion) per tablet. This composition is merely illustrative of one of many compositions within the scope of the claims.

**Table 1.**

| Component | Amount per tablet | Weight % |
|---|---|---|
| Zinc sulfate | 110 mg | 22% |
| γ-Polyglutamic acid | 110 mg | 22% |
| Microcrystalline cellulose | 100 mg | 20% |
| Starch | 85 mg | 17% |
| Silicon dioxide | 50 mg | 10% |
| Magnesium stearate | 25 mg | 5% |
| Cellulose acetate phthalate | 20 mg | 4% |
| Total | 500 mg | 100% |

### Example 2: Method of Preparing a γ-Polyglutamic acid-Zinc Composition

Coated tablets with the composition shown in Example 1 are prepared using a wet granulation technique. First, zinc sulfate and γ-polyglutamic acid are mixed together dry. Microcrystalline cellulose, starch, and silicon dioxide are further added, and the dry components are all further mixed together. The mixed components are transferred to a granulator and an appropriate amount of aqueous ethanol is added and granulation is carried out. The obtained granulated mixture is dried at 50-70°C to yield a granulated composition with less than about 5% water content. Magnesium stearate is added to and mixed with the granulated composition. The obtained mixture is compressed into tablets. Finally, the tablets are coated with cellulose acetate phthalate using standard techniques, as known to those skilled in the art.

### Example 3: γ-Polyglutamic acid-Zinc Solid Composition

The composition of an exemplary embodiment of the invention is shown in Table 2. The formulation provides 30 mg of Zn (Zn²⁺ ion) per tablet. This composition is merely illustrative of one of many compositions within the scope of the claims.

**Table 2.**

| Component - Tablet core | Amount per tablet | Weight % |
|---|---|---|
| Zinc sulfate·7H2O | 132.3 mg | 26.5% |
| γ-PGA (MW(Mn) ≤ 100 kDa) | 132.3 mg | 26.5% |
| Microcrystalline cellulose | 102.5 mg | 20.5% |
| HPMC-P | 65.0 mg | 13% |
| Maltodextrin | 37.9 mg | 7.6% |
| Carboxymethylcellulose-Ca | 5.0 mg | 1.0% |
| Aerosil^{®} | 5.0 mg | 1.0% |
| Magnesium stearate | 5.0 mg | 1.0% |
| 70% Ethanol | q.s | NA* |
| Purified water | q.s | NA* |
| SUBTOTAL | 485 mg | |

| Component - Tablet coating | Amount | Weight % |
|---|---|---|
| HPMC-P | 10.0 mg | 2.0% |
| HPMC | 5.0 mg | 1.0% |
| Isopropyl alcohol | 0.16 mL | NA* |
| Purified water | 0.13 mL | NA* |
| TOTAL | 500 mg | 100% |

| | | |
|---|---|---|
| * It is assumed here that the solvents (ethanol, isopropyl alcohol, and water) are present in insignificant amounts in the formulated tablet. | | |

### Example 4: Method of Preparing a γ-Polyglutamic acid-Zinc Solid Composition

Coated tablets with the composition shown in Example 3 were prepared as follows. First, zinc sulfate, γ-polyglutamic acid, microcrystalline cellulose, HPMC-P (hydroxypropylmethylcellulose phthalate), maltodextrin, and carboxymethylcellulose-calcium were mixed together dry. The mixed components were transferred to a granulator and an appropriate amount of 70% aqueous ethanol was added and wet granulation was carried out. The obtained granulated mixture was dried at up to about 60°C to yield a granulated composition with less than about 3% LCD (loss on drying). Silica (e.g., Aerosil^{®}) and magnesium stearate was added to and mixed with the granulated composition. The obtained mixture was compressed into tablets. The tablets were first coated using an isopropyl alcohol solution of HPMC-P, and then coated in a second step using an aqueous solution of HPMC, using standard techniques, as known to those skilled in the art.

### Example 5: γ-Polyglutamic acid-Zinc Liquid Composition

The composition of an exemplary embodiment of the invention is shown in Table 3. The formulation provides 0.68 mg of Zn (Zn²⁺ ion) per 100 g of liquid formulation. This composition is merely illustrative of one of many compositions within the scope of the claims.

**Table 3.**

| | |
|---|---|
| Suspended Solid Components | Amount |
| Zinc sulfate·7H2O | 3.011 mg |
| γ-PGA (MW(Mn) ≤ 100 kDa) | 6.848 mg |
| Sucrose | 9.5107 g |
| HPMC-P | 0.3804 g |
| Wax | 98.91 mg |
| SUBTOTAL | 10g |

| Solution Components | Amount |
|---|---|
| Xanthan gum | 0.3 g |
| Guar gum | 0.3 g |
| Xylitol | 10 g |
| Citric acid | 0.5 g |
| Limonene | 0.1 g |
| Potassium sorbate | 0.025 g |
| Water | 78.7 mL |
| TOTAL | 99.925 g |

### Example 6: Method of Preparing a γ-Polyglutamic acid-Zinc Liquid Composition

1. Preparation of coated ZnPGA microspheres (cZPM). 200 mL water containing 10 g sucrose (5% w/v), 45 mg γ-PGA, and 19.79 mg zinc sulfate heptahydrate (4.5 mg as elemental Zn) was prepared and freeze-dried. The resulting powder was then triturated in a 1:4 ratio with finely divided sucrose containing up to 5% cornstarch and pressed through a No. 50 U.S. Standard stainless steel sieve (48 Mesh). This powder was then suspended in 200 mL of white paraffin oil in a 400 mL beaker. The mixture was dispersed by stirring at 260 rpm with a 44 mm polyethylene three-blade paddle fitted to a high-torque stirrer (Type RXR1, Caframo, Wiarton, Ontario). To the suspension was added 20 mL of 10% (w/v) hydroxypropylmethylcellulose-phthalate (HPMC-P) in acetone-95% ethanol (9:1). Stirring was continued for 5 min, whereby microspheres form, and then 75 mL of chloroform was added. The suspending medium was decanted, and the microspheres were briefly resuspended in 75 mL of chloroform, and air-dried at ambient temperature. Upon drying, the microspheres were coated with Carnauba wax. Specifically, 1 g of Carnauba wax was dissolved in 200 mL of white paraffin oil at 70°C, and cooled to less than 45°C. To this cooled wax-paraffin solution, the prepared microspheres were added and suspended for 15 mins with constant stirring. The wax solution was then decanted, and the microspheres were collected on filter paper to absorb the excess wax solution to obtain coated ZnPGA microspheres (cZPM).
2. Preparation of liquid suspension solution of coated ZnPGA microspheres (cZPM). The following components: 0.3 g xanthan gum (e.g., as a suspending polymer); 0.3 g guar gum (e.g., as a viscosity agent); 10 g xylitol (e.g., as a sweetener); 0.5 g citric buffer (e.g., as a buffer); 0.1 g limonene (e.g., as a flavoring agent); 0.025 g potassium sorbate (e.g., as a preservative), were dissolved in 78.7 mL water. The pH of the aqueous solution was adjusted to pH 4.5, and then 10 g cZPM was suspended in the aqueous solution to obtain the cZPM liquid suspension.

The foregoing invention having been described in detail and by way of example and illustration, those of skill in the art will appreciate the range of compositions and methods disclosed herein and embraced by the claims.

## Claims

1. A composition for administering zinc, formulated as a solid dosage form for oral administration, comprising about 10 - 40 wt% of γ-polyglutamic acid having a number average molecular weight in the range of about 5 kDa to about 300 kDa, a zinc salt wherein the amount of zinc present per solid dosage form is about 1 mg to about 100 mg, and a gastro-resistant material.

2. The composition according to claim 1, wherein the γ-polyglutamic acid has a number average molecular weight in the range of about 50 kDa to about 100 kDa.

3. The composition according to claim 1 or 2, wherein the zinc is present in the amount of about 1 mg to about 50 mg per solid dosage form.

4. The composition according to any of claims 1-3, wherein said zinc salt is a nutritionally acceptable zinc salt.

5. The composition according to any of claims 1-4, wherein said zinc salt is selected from zinc chloride, zinc sulfate, zinc citrate, zinc acetate, zinc picolinate, zinc gluconate, amino acid-zinc chelates, and combinations thereof.

6. The composition according to any of claims 1-5, wherein said solid dosage form is a tablet or a capsule.

7. The composition according to any of claims 1-6, wherein said gastro-resistant material is selected from cellulose acetate phthalate, cellulose acetate succinate, cellulose acetate trimellitate, a copolymer of two or more monomers selected from (i) an acrylate ester, (ii) a methylacrylate ester, and (iii) methacrylic acid, polyvinyl acetate phthalate, hypromellose acetate succinate, hypromellose phthalate, sodium alginate, shellac, zein, and combinations thereof, and is included in said composition as a gastro-resistant binder or as a gastro-resistant outer coating.

8. A method for preparing a solid dosage form comprising γ-polyglutamic acid, a zinc salt, and a gastro-resistant outer coating, said method comprising:
(a) mixing together γ-polyglutamic acid having a number average molecular weight in the range of about 5 kDa to about 300 kDa, the zinc salt, wherein the amount of said zinc salt provides about 1 mg to about 75 mg of zinc per solid dosage form, one or more filler, one or more binder, and optionally one or more disintegrant;
(b) granulating the mixture obtained in step (a) to obtain a granulated mixture;
(c) mixing a lubricating agent and optionally a glidant with the granulated mixture obtained in step (b);
(d) tableting the mixture obtained in step (c) to obtain tablets;
(e) coating the tablets obtained in step (d) with a gastro-resistant outer coating,
whereby the solid dosage form is obtained.

9. The method according to claim 8, wherein the γ-polyglutamic acid has a number average molecular weight in the range of about 50 kDa to about 100 kDa.

10. The method for preparing a solid dosage form according to claim 8 or 9, wherein said granulating step is a wet granulation process using aqueous ethanol as the solvent to obtain wet granules; and the method further comprises drying the wet granules to obtain the granulated mixture with less than about 10 wt% water content.

11. A method for preparing a solid dosage form comprising γ-polyglutamic acid, a zinc salt, and a gastro-resistant binder, said method comprising:
(a) mixing together γ-polyglutamic acid having a number average molecular weight in the range of about 5 kDa to about 300 kDa, the zinc salt, wherein the amount of said zinc salt provides about 1 mg to about 75 mg of zinc per solid dosage form, the gastro-resistant binder, one or more filler, optionally one or more binder other than the gastro-resistant binder, and optionally one or more disintegrant;
(b) granulating the mixture obtained in step (a) to obtain a granulated mixture;
(c) mixing a lubricating agent and optionally a glidant with the granulated mixture obtained in step (b);
(d) tableting the mixture obtained in step (c) to obtain tablets;
(e) optionally coating the tablets obtained in step (d), whereby the solid dosage form is obtained.

12. The method according to claim 11, wherein the γ-polyglutamic acid has a number average molecular weight in the range of about 50 kDa to about 100 kDa.

13. The method for preparing a solid dosage form according to claim 11 or 12, wherein said granulating step is a wet granulation process using aqueous ethanol as the solvent to obtain wet granules; and the method further comprises drying the wet granules to obtain the granulated mixture with less than about 10 wt% water content.

14. A composition for administering zinc, formulated as a solid dosage form for oral administration, comprising γ-polyglutamic acid having a number average molecular weight in the range of about 5 kDa to about 300 kDa, a zinc salt wherein the amount of zinc present per solid dosage form is about 1 mg to about 75 mg, and means for delivering said γ-polyglutamic acid and said zinc salt to the intestine.

15. The composition according to claim 14, wherein the γ-polyglutamic acid has a number average molecular weight in the range of about 50 kDa to about 100 kDa.

16. The composition according to claim 14 or 15, further comprising one or more excipients selected from fillers and/or binders and/or disintegrants.

17. A composition according to any of claims 1 to 7 for use as a medicament in the treatment of zinc deficiency or zinc inadequacy.

18. A composition for administering zinc, formulated as a liquid suspension dosage form for oral administration, comprising about 0.01 - 10 wt% of γ-polyglutamic acid having a number average molecular weight in the range of about 5 kDa to about 500 kDa, a zinc salt wherein the amount of zinc present is about 0.001 wt% to about 10 wt%, and a gastro-resistant binder.

19. The composition according to claim 18, wherein the γ-polyglutamic acid has a number average molecular weight in the range of about 50 kDa to about 100 kDa.

20. The composition according to claim 18 or 19, wherein said zinc salt is a nutritionally acceptable zinc salt.

21. The composition according to any of claims 18 - 20, wherein said gastro-resistant binder is selected from cellulose acetate phthalate, cellulose acetate succinate, cellulose acetate trimellitate, a copolymer of two or more monomers selected from (i) an acrylate ester, (ii) a methylacrylate ester, and (iii) methacrylic acid, polyvinyl acetate phthalate, hypromellose acetate succinate, hypromellose phthalate, sodium alginate, shellac, zein, and combinations thereof.

22. A method for preparing a liquid suspension dosage form comprising γ-polyglutamic acid, a zinc salt, and a gastro-resistant binder, said method comprising:
(a) mixing together γ-polyglutamic acid having a number average molecular weight in the range of about 5 kDa to about 500 kDa, the zinc salt, wherein the amount of said zinc salt provides about 0.001 wt% to about 10 wt% of zinc in the liquid suspension, the gastro-resistant binder, optionally one or more filler, optionally one or more binder, and optionally one or more disintegrant;
(b) granulating the mixture obtained in step (a) to obtain a granulated mixture;
(c) suspending the granulated mixture obtained in step (b) in a liquid suitable for ingestion and having a pH less than about 6, whereby the liquid suspension dosage form is obtained.

23. The method according to claim 22, wherein the γ-polyglutamic acid has a number average molecular weight in the range of about 50 kDa to about 100 kDa.

24. The method for preparing a liquid suspension dosage form according to claim 22 or 23, wherein said granulating step is a wet granulation process using aqueous ethanol as the solvent to obtain wet granules; and the method further comprises drying the wet granules to obtain the granulated mixture with less than about 10 wt% water content.

25. The method according to any of claims 22, 23, or 24, wherein said liquid suspension dosage form further comprises thickening agents or viscosity enhancers to suspend the granulated mixture sufficiently to facilitate ingestion.

26. A composition according to any of claims 18 to 21 for use as a medicament in the treatment of zinc deficiency or zinc inadequacy.

## Patentansprüche

1. Zusammensetzung zur Verabreichung von Zink, formuliert als feste Darreichungsform zur oralen Verabreichung, umfassend etwa 10-40 Gew.-% γ-Polyglutaminsäure, die ein zahlenmittleres Molekulargewicht im Bereich von etwa 5 kDa bis etwa 300 kDa aufweist, ein Zinksalz, wobei die Menge an Zink, die pro fester Darreichungsform vorhanden ist, etwa 1 mg bis etwa 100 mg beträgt, und ein magensaftresistentes Material.

2. Zusammensetzung nach Anspruch 1, wobei die γ-Polyglutaminsäure ein zahlenmittleres Molekulargewicht im Bereich von etwa 50 kDa bis etwa 100 kDa aufweist.

3. Zusammensetzung nach Anspruch 1 oder 2, wobei das Zink in einer Menge von etwa 1 mg bis etwa 50 mg pro fester Darreichungsform vorhanden ist.

4. Zusammensetzung nach einem der Ansprüche 1-3, wobei das Zinksalz ein ernährungsphysiologisch annehmbares Zinksalz ist.

5. Zusammensetzung nach einem der Ansprüche 1-4, wobei das Zinksalz ausgewählt ist aus Zinkchlorid, Zinksulfat, Zinkcitrat, Zinkacetat, Zinkpicolinat, Zinkgluconat, Aminosäure-Zinkchelaten und Kombinationen davon.

6. Zusammensetzung nach einem der Ansprüche 1-5, wobei die feste Darreichungsform eine Tablette oder eine Kapsel ist.

7. Zusammensetzung nach einem der Ansprüche 1-6, wobei das magensaftresistente Material ausgewählt ist aus Celluloseacetatphthalat, Celluloseacetatsuccinat, Celluloseacetattrimellitat, einem Copolymer aus zwei oder mehr Monomeren, ausgewählt aus (i) einem Acrylatester, (ii) einem Methylacrylatester, und (iii) Methacrylsäure, Polyvinylacetatphthalat, Hypromelloseacetatsuccinat, Hypromellosephthalat, Natriumalginat, Schellack, Zein und Kombinationen davon, und in der Zusammensetzung als magenresistentes Bindemittel oder als magensaftresistente äußere Beschichtung eingeschlossen ist.

8. Verfahren zur Herstellung einer festen Darreichungsform, die γ-Polyglutaminsäure, ein Zinksalz und eine magensaftresistente äußere Beschichtung umfasst, wobei das Verfahren Folgendes umfasst:
(a) Zusammenmischen von γ-Polyglutaminsäure, die ein zahlenmittleres Molekulargewicht im Bereich von etwa 5 kDa bis etwa 300 kDa aufweist, dem Zinksalz, wobei die Menge des Zinksalzes etwa 1 mg bis etwa 75 mg Zink pro fester Darreichungsform bereitstellt, einem oder mehreren Füllstoffen, einem oder mehreren Bindemitteln und optional einem oder mehreren Desintegrationsmitteln;
(b) Granulieren der in Schritt (a) erhaltenen Mischung, um eine granulierte Mischung zu erhalten;
(c) Mischen eines Schmiermittels und optional eines Gleitmittels mit der in Schritt (b) erhaltenen granulierten Mischung;
(d) Tablettieren der in Schritt (c) erhaltenen Mischung, um Tabletten zu erhalten;
(e) Überziehen der in Schritt (d) erhaltenen Tabletten mit einem magensaftresistenten äußeren Überzug, wodurch die feste Darreichungsform erhalten wird.

9. Verfahren nach Anspruch 8, wobei die γ-Polyglutaminsäure ein zahlenmittleres Molekulargewicht im Bereich von etwa 50 kDa bis etwa 100 kDa aufweist.

10. Verfahren zur Herstellung einer festen Darreichungsform nach Anspruch 8 oder 9, wobei der Granulierschritt ein Nassgranulierverfahren unter Verwendung von wässrigem Ethanol als Lösungsmittel ist, um nasses Granulat zu erhalten; und das Verfahren weiter das Trocknen des nassen Granulats umfasst, um die granulierte Mischung mit weniger als etwa 10 Gew.-% Wassergehalt zu erhalten.

11. Verfahren zur Herstellung einer festen Darreichungsform, die γ-Polyglutaminsäure, ein Zinksalz und ein magensaftresistentes Bindemittel umfasst, wobei das Verfahren Folgendes umfasst:
(a) Zusammenmischen von γ-Polyglutaminsäure mit einem zahlenmittleren Molekulargewicht im Bereich von etwa 5 kDa bis etwa 300 kDa, dem Zinksalz, wobei die Menge des Zinksalzes etwa 1 mg bis etwa 75 mg Zink pro fester Darreichungsform aufweist, dem magensaftresistenten Bindemittel, einem oder mehreren Füllstoffen, optional einem oder mehreren anderen Bindemitteln als dem magensaftresistenten Bindemittel, und optional einem oder mehreren Desintegrationsmitteln;
(b) Granulieren der in Schritt (a) erhaltenen Mischung, um eine granulierte Mischung zu erhalten;
(c) Mischen eines Schmiermittels und optional eines Gleitmittels mit der in Schritt (b) erhaltenen granulierten Mischung;
(d) Tablettieren der in Schritt (c) erhaltenen Mischung, um Tabletten zu erhalten;
(e) optional Überziehen der in Schritt (d) erhaltenen Tabletten, wodurch die feste Darreichungsform erhalten wird.

12. Verfahren nach Anspruch 11, wobei die γ-Polyglutaminsäure ein zahlenmittleres Molekulargewicht im Bereich von etwa 50 kDa bis etwa 100 kDa aufweist.

13. Verfahren zur Herstellung einer festen Dosierungsform nach Anspruch 11 oder 12, wobei der Granulierschritt ein Nassgranulierverfahren unter Verwendung von wässrigem Ethanol als Lösungsmittel ist, um nasses Granulat zu erhalten; und das Verfahren weiter das Trocknen des nassen Granulats umfasst, um die granulierte Mischung mit weniger als etwa 10 Gew.-% Wassergehalt zu erhalten.

14. Zusammensetzung zur Verabreichung von Zink, formuliert als feste Darreichungsform zur oralen Verabreichung, umfassend γ-Polyglutaminsäure, die ein zahlenmittleres Molekulargewicht im Bereich von etwa 5 kDa bis etwa 300 kDa aufweist, ein Zinksalz, wobei die Menge an Zink, die pro fester Darreichungsform vorliegt, etwa 1 mg bis etwa 75 mg beträgt, und Mittel zur Abgabe der γ-Polyglutaminsäure und des Zinksalzes an den Darm.

15. Zusammensetzung nach Anspruch 14, wobei die γ-Polyglutaminsäure ein zahlenmittleres Molekulargewicht im Bereich von etwa 50 kDa bis etwa 100 kDa aufweist.

16. Zusammensetzung nach Anspruch 14 oder 15, weiter umfassend einen oder mehrere Hilfsstoffe, ausgewählt aus Füllstoffen und/oder Bindemitteln und/oder Desintegrationsmitteln.

17. Zusammensetzung nach einem der Ansprüche 1 bis 7 zur Verwendung als Arzneimittel bei der Behandlung von Zinkmangel oder Zinkunzulänglichkeit.

18. Zusammensetzung zur Verabreichung von Zink, formuliert als flüssige Suspensionsdosierungsform zur oralen Verabreichung, umfassend etwa 0,01 - 10 Gew.-% γ-Polyglutaminsäure mit einem zahlenmittleren Molekulargewicht im Bereich von etwa 5 kDa bis etwa 500 kDa, ein Zinksalz, wobei die Menge an vorhandenem Zink etwa 0,001 Gew.-% bis etwa 10 Gew.-% beträgt, und ein magensaftresistentes Bindemittel.

19. Zusammensetzung nach Anspruch 18, wobei die γ-Polyglutaminsäure ein zahlenmittleres Molekulargewicht im Bereich von etwa 50 kDa bis etwa 100 kDa aufweist.

20. Zusammensetzung nach Anspruch 18 oder 19, wobei das Zinksalz ein ernährungsphysiologisch annehmbares Zinksalz ist.

21. Zusammensetzung nach einem der Ansprüche 18-20, wobei das magensaftresistente Bindemittel ausgewählt ist aus Celluloseacetatphthalat, Celluloseacetatsuccinat, Celluloseacetattrimellitat, einem Copolymer aus zwei oder mehr Monomeren, ausgewählt aus (i) einem Acrylatester, (ii) einem Methylacrylatester, und (iii) Methacrylsäure, Polyvinylacetatphthalat, Hypromelloseacetatsuccinat, Hypromellosephthalat, Natriumalginat, Schellack, Zein und Kombinationen davon ausgewählt sind.

22. Verfahren zur Herstellung einer flüssigen Suspensionsdarreichungsform, die γ-Polyglutaminsäure, ein Zinksalz und ein magensaftresistentes Bindemittel umfasst, wobei das Verfahren umfasst:
(a) Zusammenmischen von γ-Polyglutaminsäure mit einem zahlenmittleren Molekulargewicht im Bereich von etwa 5 kDa bis etwa 500 kDa, dem Zinksalz, wobei die Menge des Zinksalzes etwa 0,001 Gew.-% bis etwa 10 Gew.-% Zink in der flüssigen Suspension aufweist, dem magensaftresistenten Bindemittel, optional einem oder mehreren Füllstoffen, optional einem oder mehreren Bindemitteln und optional einem oder mehreren Desintegrationsmitteln;
(b) Granulieren der in Schritt (a) erhaltenen Mischung, um eine granulierte Mischung zu erhalten;
(c) Suspendieren der in Schritt (b) erhaltenen granulierten Mischung in einer zur Einnahme geeigneten Flüssigkeit, die einen pH-Wert von weniger als etwa 6 aufweist,
wodurch die flüssige Suspensionsdarreichungsform erhalten wird.

23. Verfahren nach Anspruch 22, wobei die γ-Polyglutaminsäure ein zahlenmittleres Molekulargewicht im Bereich von etwa 50 kDa bis etwa 100 kDa aufweist.

24. Verfahren zur Herstellung einer flüssigen Suspensionsdarreichungsform nach Anspruch 22 oder 23, wobei der Granulierschritt ein Nassgranulierverfahren unter Verwendung von wässrigem Ethanol als Lösungsmittel ist, um nasses Granulat zu erhalten; und das Verfahren weiter das Trocknen des nassen Granulats umfasst, um die granulierte Mischung mit weniger als etwa 10 Gew.-% Wassergehalt zu erhalten.

25. Verfahren nach einem der Ansprüche 22, 23 oder 24, wobei die flüssige Suspensionsdarreichungsform weiter Verdickungsmittel oder Viskositätsverstärker umfasst, um die granulierte Mischung ausreichend zu suspendieren, um die Einnahme zu erleichtern.

26. Zusammensetzung nach einem der Ansprüche 18 bis 21 zur Verwendung als Arzneimittel bei der Behandlung von Zinkmangel oder Zinkunzulänglichkeit.

## Revendications

1. Composition pour l'administration de zinc, formulée en tant que forme galénique solide pour administration orale, comprenant environ 10 à 40 % en poids d'acide γ-polyglutamique ayant un poids moléculaire moyen en nombre dans la plage d'environ 5 kDa à environ 300 kDa, un sel de zinc où la quantité de zinc présente par forme galénique solide est d'environ 1 mg à environ 100 mg, et un matériau gastro-résistant.

2. Composition selon la revendication 1, dans laquelle l'acide γ-polyglutamique a un poids moléculaire moyen en nombre dans la plage d'environ 50 kDa à environ 100 kDa.

3. Composition selon la revendication 1 ou la revendication 2, dans laquelle le zinc est présent en la quantité d'environ 1 mg à environ 50 mg par forme galénique solide.

4. Composition selon l'une quelconque des revendications 1 à 3, dans laquelle ledit sel de zinc est un sel de zinc acceptable sur le plan nutritionnel.

5. Composition selon l'une quelconque des revendications 1 à 4, dans laquelle ledit sel de zinc est sélectionné parmi le chlorure de zinc, le sulfate de zinc, le citrate de zinc, l'acétate de zinc, le picolinate de zinc, le gluconate de zinc, les chélates d'acide aminé-zinc, et les combinaisons de ceux-ci.

6. Composition selon l'une quelconque des revendications 1 à 5, dans laquelle ladite forme galénique solide est un comprimé ou une capsule.

7. Composition selon l'une quelconque des revendications 1 à 6, dans laquelle ledit matériau gastro-résistant est sélectionné parmi un phtalate d'acétate de cellulose, un succinate d'acétate de cellulose, un trimellitate d'acétate de cellulose, un copolymère de deux ou plus de deux monomères sélectionnés parmi (i) un ester d'acrylate, (ii) un ester d'acrylate de méthyle, et (iii) l'acide méthacrylique, un polyphtalate d'acétate de vinyle, un succinate d'acétate d'hypromellose, un phtalate d'hypromellose, l'alginate de sodium, la gomme-laque, la zéine, et des combinaisons de ceux-ci, et est inclus dans ladite composition en tant que liant gastro-résistant ou en tant qu'enrobage externe gastro-résistant.

8. Procédé de préparation d'une forme galénique solide comprenant un acide γ-polyglutamique, un sel de zinc, et un enrobage externe gastro-résistant, ledit procédé comprenant :
(a) le mélange ensemble d'un acide γ-polyglutamique ayant un poids moléculaire moyen en nombre dans la plage d'environ 5 kDa à environ 300 kDa, du sel de zinc, où la quantité dudit sel de zinc fournit environ 1 mg à environ 75 mg de zinc par forme galénique solide, d'une ou plusieurs charges, d'un ou plusieurs liants, et facultativement d'un ou plusieurs agents de délitement ;
(b) la granulation du mélange obtenu dans l'étape (a) pour obtenir un mélange granulé ;
(c) le mélange d'un agent lubrifiant et facultativement d'un agent de glissement avec le mélange granulé obtenu dans l'étape (b) ;
(d) la formation de comprimés à partir du mélange obtenu dans l'étape (c) pour obtenir des comprimés ;
(e) l'enrobage des comprimés obtenus dans l'étape (d) avec un enrobage externe gastro-résistant, grâce à quoi la forme galénique solide est obtenue.

9. Procédé selon la revendication 8, dans lequel l'acide γ-polyglutamique a un poids moléculaire moyen en nombre dans la plage d'environ 50 kDa à environ 100 kDa.

10. Procédé de préparation d'une forme galénique solide selon la revendication 8 ou la revendication 9, dans lequel ladite étape de granulation est un procédé de granulation humide utilisant de l'éthanol aqueux comme solvant pour obtenir des granulés humides ; et le procédé comprend en outre le séchage des granulés humides pour obtenir le mélange granulé avec moins d'environ 10 % en poids d'eau.

11. Procédé de préparation d'une forme galénique solide comprenant un acide γ-polyglutamique, un sel de zinc, et un liant gastro-résistant, ledit procédé comprenant :
(a) le mélange ensemble d'un acide γ-polyglutamique ayant un poids moléculaire moyen en nombre dans la plage d'environ 5 kDa à environ 300 kDa, du sel de zinc, dans lequel la quantité dudit sel de zinc fournit environ 1 mg à environ 75 mg de zinc par forme galénique solide, du liant gastro-résistant, d'une ou plusieurs charges, facultativement d'un ou plusieurs liants autres que le liant gastro-résistant, et facultativement d'un ou plusieurs agents de délitement ;
(b) la granulation du mélange obtenu dans l'étape (a) pour obtenir un mélange granulé ;
(c) le mélange d'un agent lubrifiant et facultativement d'un agent de glissement avec le mélange granulé obtenu dans l'étape (b) ;
(d) la formation de comprimés à partir du mélange obtenu dans l'étape (c) pour obtenir des comprimés ;
(e) facultativement l'enrobage des comprimés obtenus dans l'étape (d), grâce à quoi la forme galénique solide est obtenue.

12. Procédé selon la revendication 11, dans lequel l'acide γ-polyglutamique a un poids moléculaire moyen en nombre dans la plage d'environ 50 kDa à environ 100 kDa.

13. Procédé de préparation d'une forme galénique solide selon la revendication 11 ou la revendication 12, dans lequel ladite étape de granulation est un procédé de granulation humide utilisant de l'éthanol aqueux comme solvant pour obtenir des granulés humides ; et le procédé comprend en outre le séchage des granulés humides pour obtenir le mélange granulé avec moins d'environ 10 % en poids d'eau.

14. Composition pour l'administration de zinc, formulée sous la forme d'une forme galénique solide pour administration orale, comprenant un acide γ-polyglutamique ayant un poids moléculaire moyen en nombre dans la plage d'environ 5 kDa à environ 300 kDa, un sel de zinc où la quantité de zinc présente par forme galénique solide est d'environ 1 mg à environ 75 mg, et un moyen pour administrer ledit acide γ-polyglutamique et ledit sel de zinc à l'intestin.

15. Composition selon la revendication 14, dans laquelle l'acide γ-polyglutamique a un poids moléculaire moyen en nombre dans la plage d'environ 50 kDa à environ 100 kDa.

16. Composition selon la revendication 14 ou la revendication 15, comprenant en outre un ou plusieurs excipients sélectionnés parmi des charges et/ou des liants et/ou des agents de délitement.

17. Composition selon l'une quelconque des revendications 1 à 7 pour utilisation comme médicament dans le traitement d'une carence en zinc ou d'une insuffisance en zinc.

18. Composition pour l'administration de zinc, formulée en tant que forme galénique de type suspension liquide pour administration orale, comprenant environ 0,01 à 10 % en poids d'acide γ-polyglutamique ayant un poids moléculaire moyen en nombre dans la plage d'environ 5 kDa à environ 500 kDa, un sel de zinc où la quantité de zinc présente est d'environ 0,001 % en poids à environ 10 % en poids, et un liant gastro-résistant.

19. Composition selon la revendication 18, dans laquelle l'acide γ-polyglutamique a un poids moléculaire moyen en nombre dans la plage d'environ 50 kDa à environ 100 kDa.

20. Composition selon la revendication 18 ou la revendication 19, dans laquelle ledit sel de zinc est un sel de zinc acceptable sur le plan nutritionnel.

21. Composition selon l'une quelconque des revendications 18 à 20, dans laquelle ledit liant gastro-résistant est sélectionné parmi un phtalate d'acétate de cellulose, un succinate d'acétate de cellulose, un trimellitate d'acétate de cellulose, un copolymère de deux ou plus de deux monomères sélectionnés parmi (i) un ester d'acrylate, (ii) un ester d'acrylate de méthyle, et (iii) l'acide méthacrylique, un polyphtalate d'acétate de vinyle, un succinate d'acétate d'hypromellose, un phtalate d'hypromellose, l'alginate de sodium, la gomme-laque, la zéine, et des combinaisons de ceux-ci.

22. Procédé de préparation d'une forme galénique de type suspension liquide comprenant un acide y-polyglutamique, un sel de zinc, et un liant gastro-résistant, ledit procédé comprenant :
(a) le mélange ensemble d'un acide γ-polyglutamique ayant un poids moléculaire moyen en nombre dans la plage d'environ 5 kDa à environ 500 kDa, du sel de zinc, où la quantité dudit sel de zinc fournit environ 0,001 % en poids à environ 10 % en poids de zinc dans la suspension liquide, du liant gastro-résistant, facultativement d'une ou plusieurs charges, facultativement d'un ou plusieurs liants, et facultativement d'un ou plusieurs agents de délitement ;
(b) la granulation du mélange obtenu dans l'étape (a) pour obtenir un mélange granulé ;
(c) la mise en suspension du mélange granulé obtenu dans l'étape (b) dans un liquide approprié pour l'ingestion et ayant un pH inférieur à environ 6, grâce à quoi la forme galénique de type suspension liquide est obtenue.

23. Procédé selon la revendication 22, dans lequel l'acide γ-polyglutamique a un poids moléculaire moyen en nombre dans la plage d'environ 50 kDa à environ 100 kDa.

24. Procédé de préparation d'une forme galénique de type suspension liquide selon la revendication 22 ou la revendication 23, dans lequel ladite étape de granulation est un procédé de granulation humide utilisant de l'éthanol aqueux comme solvant pour obtenir des granulés humides ; et le procédé comprend en outre le séchage des granulés humides pour obtenir le mélange granulé avec moins d'environ 10 % en poids d'eau.

25. Procédé selon l'une quelconque des revendications 22, 23, ou 24, dans lequel ladite forme galénique de type suspension liquide comprend en outre des agents épaississants ou des agents améliorant la viscosité pour mettre en suspension le mélange granulé suffisamment pour faciliter l'ingestion.

26. Composition selon l'une quelconque des revendications 18 à 21 pour utilisation comme médicament dans le traitement d'une carence en zinc ou d'une insuffisance en zinc.
